# EUROPEAN PATENT SPECIFICATION

(11) **EP 3 078 360 B1**
(45) Date of publication and mention of the grant of the patent: **09.01.2019**
(21) Application number: 14868096.0
(22) Date of filing: 23.10.2014
(51) Int. Cl.: A61F 13/00

(54) **WOUND DRESSING WITH THREE-LAYER STRUCTURE AND PREPARATION METHOD THEREOF**
WUNDVERBAND MIT EINER DREISCHICHTIGEN STRUKTUR UND HERSTELLUNGSVERFAHREN DAFÜR
PANSEMENT AYANT UNE STRUCTURE À TROIS COUCHES, ET SON PROCÉDÉ DE PRÉPARATION

(30) Priority: 03.12.2013 CN 201310642677
(43) Date of publication of application: 12.10.2016
(73) Proprietor: Foshan United Medical Technologies Ltd, Guangdong 528225 (CN)
(72) Inventor: WANG, Xiaodong, Foshan Guangdong 528225 (CN); FENG, Caixia, Foshan Guangdong 528225 (CN); HE, Guibiao, Foshan Guangdong 528225 (CN); XIAO, Jianpeng, Foshan Guangdong 528225 (CN); MO, Xiaohui, Foshan Guangdong 528225 (CN)
(74) Representative: Wilson Gunn
(86) International application number: PCT/CN2014/089317
(87) International publication number: WO 2015/081769

(56) References cited:
- CN-A- 1 329 881
- CN-A- 1 358 484
- CN-A- 102 076 291
- CN-A- 103 655 046
- JP-A- 2004 073 759
- US-A1- 2004 082 925
- US-A1- 2006 020 235
- US-A1- 2010 030 170
- US-A1- 2013 012 902

## Description

### Technical field

This invention relates to a three-layered wound dressing and its method of manufacturing.

### Background art

Advanced wound management requires wound dressings with a high absorption capacity as well as a sufficient moisture retention capacity. This will make the dressing capable of providing a moist environment for optimum wound healing. The current fibrous wound dressings, such as alginate, chitosan and carboxymethyl cellulose (CMC) fibres or their blending, all have an absorbency of between 12-20 g/100cm2.

The typical example is EP 0927013B1 which disclosed a wound dressing that is made of two gelling fibres.

The base weight of these dressings is normally at 100-150 gsm, therefore their absorbency is limited, typically maximum 25 g/100cm2. This will be insufficient when it comes to a heavily exudate wound. In that case, the dressing will have to be changed more frequently. Therefore there is a need for a high absorbency wound dressing, specifically higher than 30 g/100 cm2.

Furthermore, some fibres (such as alginate, chitosan and CMC) are all hydrophilic, they have a strong wicking ability. When an area of the dressing has absorbed the fluid, the absorbed fluid will quickly spread over to other areas. This is not ideal for wound care, for example, when the dressing absorbs the wound fluid, it may allow the absorbed fluid wicked over to the areas of healthy skin, this is also called lateral wicking. The healthy skin will be damaged by the maceration of the wound fluid.

EP078378B1 designed a three-layered wound dressing. The wound contact layer has some therapeutic effect to the wound, such as blood clotting etc. The middle layer is a hydrophilic fabric layer and the outer layer is a permeable polyurethane membrane. This method can provide some therapeutic treatment to the wound but did not solve the lateral wicking problem.

EP1303239 disclosed a three layered wound dressing. The wound contact layer of the dressing is a grid coated adhesive to fix the wound onto the body. The middle layer is an absorbent layer which is also covered by the outer layer. The dressing is featured with a grid coated adhesive.

All these designs have high absorbency but do not provide the solution for lateral wicking. What is needed is vertical wicking, which can take the absorbed fluid away from the wound contact layer but not spread it laterally over to the healthy skin. Therefore there is a need for a dressing which has properties of high absorbency and vertical wicking.

### Disclosure of invention

The present invention provides a three-layered wound dressing. The dressing consists of three layers, i.e. wound contact layer, middle absorbent layer and outer layer. The wound contact layer comprises hydrophilic fibres and hydrophobic fibres. The middle absorbent layer comprises some other hydrophilic fibres and hydrophobic fibres. The outer layer comprises hydrophobic fibres.

The objectives of the wound contact layer is to absorb and transfer quickly the wound fluid to the middle absorbent layer. This transfer is almost vertical, i.e. with no or very little lateral transfer, thus ensuring the majority of the wound fluid is transferred to the middle absorbent layer, not to the surrounding areas. The middle absorbent layer is designed to absorb and to retain the wound fluid. The outer layer can be a coloured fabric so to differentiate it from the wound contact layer. Because the outer layer is a hydrophobic material, it can help to keep the wound fluid absorbed by the middle layer within the dressing.

According to the present invention, the wound contact layer contains 75% or less by weight (of the wound contact layer) hydrophilic fibres and hydrophobic fibres. The proportion of hydrophilic fibre is preferably 60% or less, most preferably 50% by weight. This is different from the design theory of some traditional dressings where the wound contact layer is made up with 100% hydrophilic fibres. With 100% hydrophilic fibre, it is difficult to avoid lateral wicking and maceration of surrounding healthy skin. The present invention has surprisingly found that by adding a certain percent of hydrophobic fibres to the dressing, the lateral wicking can be reduced or prevented. According to the present invention, the hydrophilic fibres in the wound contact layer can be selected from: viscose fibres, absorbent acrylic fibres, absorbent polypropylene fibres, Lyocell fibres, alginate fibres, chitosan fibres, carboxymethyl cellulose fibres (CMC), carboxy ethyl cellulose (CEC) fibres, acylated chitosan fibres, carboxymethyl chitosan fibres. The hydrophobic fibres in the wound contact layer can be selected from polypropylene fibres, polyester fibres, nylon (Polyamide) fibres, polyethylene fibres, hydrophobic chitosan fibres, bi-component fibres.

Amongst the above hydrophilic fibres, the viscose, lyocell, alginate, carboxymethyl cellulose, carboxy ethyl cellulose, acylatedd chitosan fibres are all well know. Chitosan fibres are normally hydrophobic, but can be made hydrophilic by adding certain surfactants or by other modifications. The same apples to acrylic and polypropylene fibres.

The difference between hydrophilic and hydrophobic fibres can be seen by a simple sink test. When the fibres are placed onto a water surface, the hydrophilic fibres will sink but the hydrophobic will not. This test can be described in details as follow:
1. Take a beaker with 200 ml of distilled water;
2. Take a fixed amount of fibre e.g. 1 gram;
3. Make fibres into a knot so they are not spread over on the water, then place it into the beaker;
4. If the fibre knot sinks within 5 seconds, it is hydrophilic otherwise hydrophobic.

Hydrophilic and hydrophobic fibres can be mixed at a certain ratio at carding stage, then opened together, followed by carding and crosslapping which will make further blending. The wound contact layer can be a needle punched nonwoven. If the hydrophobic fibres contains bi-component fibres, the fabric can also be made by passing the web into a high temperature oven or a pair of hot rollers which will make the bi-component fibre partially melt causing the interlink between the bi-component fibres.

The wound contact layer can be bonded with the other two layers by needle punching, it can also be made by adhesive or heat laminating. This is very much dependent on the handling requirement of the finished dressing and whether the dressing contains fibres with a low melting point. If the dressing contains fibres with a low melting point, the lamination can be made with any of three methods (needling, heat or adhesive). If the dressing dos not contain fibres with low melt point, the lamination can only be made with needling and adhesive. In general, the heat lamination and adhesive lamination will make the finished dressing feel rigid and hard (harsh handling). If softness is one of the key requirements, needle punching is the best way for lamination of three layers.

It is also possible to laminate the wound contact layer directly onto the other two layers after blending, opening and carding.

Accordingly to the present invention, the weight of the wound contact layer is between 50-500 g/m2 (gsm), preferably 80-450 gsm, most preferably 100-400 gsm.

Accordingly to the present invention, the, middle layer also contains hydrophilic and hydrophobic layer. The percentage of hydrophobic fibres shall be 75% or less (by weight of the middle layer), preferably 60% or less, most preferably 50% or less. The main objective of the middle layer is to absorb wound exudates transferred from the wound contact layer, therefore one of the key characteristics of the present invention is the high absorbency of the middle layer. The ratio between the hydrophilic fibres to the hydrophobic fibres in the middle layer shall be selected according to the requirements of the finished dressing. With some hydrophobic fibres in the middle layer, it can reduce some gelling fibres forming gel blocks which may prevent the spreading of the fluid along the middle layer thus limiting the absorption capacity of the layer. However too much hydrophobic fibre will reduce the absorption capacity of the middle layer.

Accordingly to the present invention, the hydrophilic fibres on the middle layer can be selected from absorbent chitosan fibres, viscose fibres, Lyocell fibres, alginate fibres, carboxymethyl cellulose fibres, carboxy ethyl cellulose fibres, acylated chitosan fibres, carboxymethyl chitosan fibres, cross-linked acrylates copolymer super absorbent fibres, wood pump. The hydrophobic fibres can be selected from polypropylene fibres, polyester fibres, nylon (Polyamide) fibres, polyethylene fibres, hydrophobic chitosan fibres, bi-component fibres.

The above hydrophilic fibres have their own characteristics such as carboxymethyl cellulose fibres, carboxy ethyl cellulose fibres, acylated chitosan fibres, carboxymethyl chitosan fibres and cross-linked acrylates copolymer super absorbent fibres (SAF) are all very absorbent. The alginate fibres can release calcium ions, the chitosan fibres are bacteriostatic, and the viscose fibres have high wet strength.

The middle layer can be made into fabric first before laminating to the layers. It can also be made into web then laminated to the outer layer directly.

Accordingly to the present invention, the weight of the middle layer shall be between 80-500 gsm, preferably 100-450 gsm, most preferably 100-400 gsm.

Accordingly to the present invention, the outer layer is made with hydrophobic fibres and can be selected from polypropylene fibres, polyester fibres, nylon (Polyamide) fibres, polyethylene fibres, hydrophobic chitosan fibres, bi-component fibres. The bi-component fibres in the present invention can be PE/PP fibres, or Nylon (Polyamide)/PE, or PET/Nylon (Polyamide).

Most hydrophobic fibres are made with hot melt method, therefore the outer layer of the present invention is usually made with hot melt spinning or spunbond methods. Forthose fibres that are not made by hot melt method, it can also be made with hydro entanglement method.

Accordingly to the present invention, the weight of the outer layer shall be between 10-100 gsm, preferably 10-80 gsm, most preferably 10-60 gsm.

Accordingly to the present invention, the wound contact layer, middle layer and the outer layer can be coloured or not coloured (original color of the fibre). In general the outer layer is coloured and the wound contact layer is not coloured so that the end user (such as nurse or doctor) can tell the outer layer and the wound contact layer. The middle layer is usually not coloured.

Accordingly to the present invention, the dressing of the present invention can be made into antimicrobial by adding some antimicrobial agents such as silver, silver compound, silver complex, Polyhexamethylene biguanide (PHMB) and honey etc can be added to hydrophilic fibres and/or hydrophobic fibres in the wound contact layer and/or middle layer.

The present invention does not limit the method of adding antimicrobial agents. These methods include:
- Adding antimicrobial agents such as silver nitrate, silver chloride, silver carbonate, silver sodium zirconium hydrogen phosohate into polymer solution of fibre spinning (dope), thus making the fibres antimicrobial. This method can make the silver content in the fibre between 0.001%-10%.
- Spraying the silver solution (such as nano silver solution) onto the fibre surface.

This method can make the silver content of the fibre between 0.01% - 2%.
- Coating the silver onto the fibre surface. This method can make the silver content of the fibre between 0.1% - 25%.

The present invention also discloses a method of manufacturing the above dressing. The main elements of the method contain the following
1. Manufacturing the wound contact layer, middle layer and the outer layer separately using hot melt, needle punch or hydro entanglement methods. Then by heat lamination or needle punching or adhesive laminate the wound contact layer, middle layer and the outer layer together, followed by slitting, cutting, packaging and sterilization.
2. Manufacturing the wound contact layer and the middle layer separately using hot melt, needle punch or hydro entanglement methods. Then by heat lamination or needle punching or adhesive laminate the wound contact layer and the middle layer together, then manufacture the outer layer directly onto the laminated layers (wound contact and middle layers), followed by slitting, cutting, packaging and sterilization
3. Manufacturing the middle layer and the outer layer separately using hot melt, needle punch or hydro entanglement methods. Then by heat lamination or needle punching or adhesive laminate the middle layer and the outer layer together, then manufacture the wound contact layer directly onto the laminated layers (middle and outer layers), followed by slitting, cutting, packaging and sterilization.

The wound dressing in the present invention can transfer the fluid vertically (other than laterally). When the wound contact layer absorbs the fluid, it can transfer the wound exudates into the middle layer quickly and keep the wound contact layer relevantly dry, preventing the maceration of the surrounding healthy skin. The dressing can be used for the management of chronic wounds particularly the heavily exudating wounds.

### Drawings

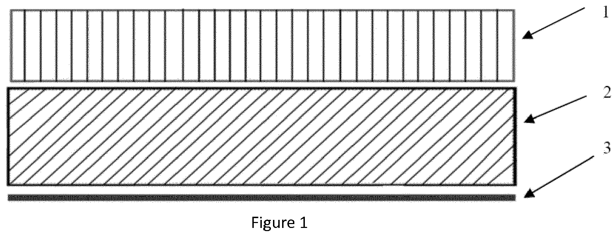

Fig.1: The structure of the three-layered wound dressing.
Where 1 is the wound contact layer, 2 is the middle layer and 3 is the outer layer The present invention can be illustrated by the following methods.

### Example 1

The three layers are made separately in the following method.

Wound contact layer: contains 75% by weight of polypropylene fibres (PP), fibre linear density 2.0 dtex, fibre length 38 mm; and 25% of alginate fibres, fibre linear density 2.5 dtex, fibre length 75 mm. The above two fibres are mixed evenly and then processed by opening, carding, crosslapping and needle punching. The needling density is 80/cm2. The finished fabric has a weight of 310 gsm.

The middle layer: contains 35% by weight of cross-linked acrylates copolymer super absorbent fibres (SAF), fibre linear density 2.7 dtex, length 10 mm; 50% by weight of wood pump, fibre linear density 2.5 dtex, length 10 mm; and 15% by weight of PP/PE bi-component fibres, fibre linear density 2.0 dtex, length 10 mm. All three fibres are blended evenly, followed by opening and air laying and finally hot oven to form the fabric. The weight of the fabric is 180 gsm.

The outer layer: PP spun bond fabric, weight 30 gsm, beige colour.

The heat lamination method is used to combine the middle layer and the outer layer, then the needle punch method is used to laminate the wound contact layer onto the above laminated layers.

Samples are cut into 10x10 cm, then packed into pouches, and sterilized by gamma irradiation. The finished dressing has a weight of 520 gsm, and an absorbency of 44 g/100 cm2.

### Example 2

The three layers are made in the following method.

Wound contact layer: contains 50% by weight of hydrophobic chitosan fibres, fibre linear density 2.2 dtex, fibre length 51 mm; and 50% by weight of viscose fibres, fibre linear density 1.7 dtex, and fibre length 51 mm. The above two fibres are mixed evenly and then processed by opening, carding, crosslapping and needle punching. The needling density is 100/cm2. The weight of the finished fabric is 100 gsm.

The middle layer: contains 35% by weight of SAF fibres, fibre linear density 2.7 dtex, length 25 mm; 15% by weight of viscose fibres, fibre linear density 2.5 dtex, length 51 mm; and 50% by weight of PP fibres, fibre linear density 2.0 dtex, and length 38 mm. All three fibres are blended evenly, followed by opening, carding, cross-lapping and needle punched into nonwoven fabric. The needling density is 100/cm2. The weight of the fabric is 150 gsm.

The outer layer: hydro entangled Lyocell nonwoven, weight 40 gsm, white colour. The wound contact layer, middle layer and the outer layer are placed onto the conveyor belt of the cross-lapping machine, and fed into the needle loom and needled into laminated fabric. The needle density is 150/cm2.

Samples are cut into 10x10 cm, then packed into pouches and sterilized by EtO. The finished dressing has a weight of 290 gsm, absorbency 47 g/100 cm2.

### Example 3

The three layers are made in the following method.

Wound contact layer: contains 10% by weight of hydrophobic chitosan fibres, fibre linear density 2.2 dtex, fibre length 51 mm; and 75% by weight of viscose fibres, fibre linear density 1.7 dtex, fibre length 51 mm; and 15% by weight of PP/PE bi-component fibres, fibre linear density 2.0 dtex, length 38 mm. The above three fibres are mixed evenly and then processed by opening, carding, and then heat processing to form the fabric. The weight of the finished fabric is 300 gsm.

The middle layer: contains 20% by weight of alginate fibres, fibre linear density 2.5 dtex, length 75 mm; 50% by weight of Lyocell fibres, fibre linear density 1.7 dtex, length 51 mm; and 30% by weight of PP/PE bi-component fibres, fibre linear density 2.0 dtex, length 38 mm. All three fibres are blended evenly, followed by opening, carding, and heat process to form the nonwoven fabric. The weight of the fabric is 150 gsm.

The outer layer: PP spun bond fabric, weight 30 gsm, pink colour.

The wound contact layer, middle layer and the outer layer are placed together into a heat process to laminate the three layers.

Samples are cut into 10x10 cm, then packed into pouches and sterilized by EtO. The finished dressing has a weight of 480 gsm, absorbency 35 g/100 cm2.

### Example 4

The three layers are made in the following method.

Wound contact layer: contains 70% by weight of polypropylene fibres (PP), fibre linear density 2.0 dtex, fibre length 38 mm; and 30% of Lyocell fibres, fibre linear density 1.7 dtex, fibre length 51 mm. The above two fibres are mixed evenly and then processed by opening, carding and crosslapping.

The middle layer: contains 25% by weight of CMC fibres, fibre linear density 2.2 dtex, length 50 mm; 75% by weight of PET fibres, fibre linear density 1.4 dtex, length 51 mm. All two fibres are blended evenly, followed by opening, carding, crosslapping and needle punching. The weight of the needled fabric is 200 gsm.

The outer layer: PP spun bond fabric, weight 30 gsm, beige colour.

During the process to manufacture the wound contact layer, the middle layer and the outer layer are placed in order (outer layer at the bottom) onto the conveyor belt of the cross lapping machine, then fed into the needling looms. The needling density was 150/cm2. The needling has pushed the fibres of the wound contact layer through the middle layer and then into the outer layer, thus laminating the three layers together. Samples are cut into 10x10 cm, then packed into pouches, and sterilized by gamma irradiation. The finished dressing has a weight of 430 gsm, absorbency 39 g/100 cm2.

### Example 5

The three layers are made in the following method.

Wound contact layer: contains 75% by weight of polypropylene fibres (PP), fibre linear density 2.0 dtex, fibre length 38 mm; and 25% of silver alginate fibres, silver content 2.1%, fibre linear density 2.6 dtex, fibre length 75 mm. The above two fibres are mixed evenly and then processed by opening, carding, crosslapping and needle punching. The needling density is 80/cm2. The weight of the finished fabric is 300 gsm.

The middle layer: contains 25% by weight of SAF fibres, fibre linear density 2.7 dtex, length 38 mm; 75% by weight of PET fibres, fibre linear density 1.4 dtex, length 51 mm. The two fibres are blended evenly, followed by opening, carding, cross-lapping and needle punched into nonwoven fabric. The weight of the fabric is 200 gsm.

The outer layer: PP spun bond fabric, weight 30 gsm, beige colour.

The wound contact layer, middle layer and the outer layer are placed onto the conveyor belt of the cross-lapping machine, and fed into the needle loom and needled into laminated fabric. The needle density is 150/cm2.

Samples are cut into 10x10 cm, then packed into pouches and sterilized by Gamma. The finished dressing has a weight of 530 gsm, absorbency 42 g/100 cm2.

### Example 6

The three layers are made in the following method.

Wound contact layer: contains 70% by weight of polypropylene fibres (PP), fibre linear density 2.0 dtex, fibre length 38 mm; and 30% of Lyocell fibres, fibre linear density 1.7 dtex, fibre length 51 mm. The above two fibres are mixed evenly and then processed by opening, carding, crosslapping and needle punching. The needling density is 80/cm2, the weight of the finished layer is 100 gsm..

The middle layer: contains 40% by weight of silver alginate fibres, silver content 2.1%, fibre linear density 2.6 dtex, length 75 mm; 60% by weight of PET fibres, fibre linear density 1.4 dtex, length 51 mm. All two fibres are blended evenly, followed by opening, carding, crosslapping and needle punching. The weight of the needled fabric is 100 gsm.

The outer layer: PP spun bond fabric, weight 30 gsm, beige colour.

The wound contact layer, middle layer and the outer layer are placed onto the conveyor belt of the cross-lapping machine, and fed into the needle loom and needled into laminated fabric. The needle density is 150/cm2.

Samples are cut into 10x10 cm, then packed into pouches, and sterilized by gamma irradiation. The finished dressing has a weight of 230 gsm, absorbency 32 g/100 cm2.

### Example 7

The wound contact layer and the middle layer are the same as the ones from Example 6, feed both fabrics into the needle loom to laminate by needling. The laminated fabric is then placed before the calender rollers of the polypropylene spun bond line, ensuring the middle layer facing the income polypropylene material so that spun bond polypropylene is laid onto the middle layer, and all three layers are fed into the calender rollers together, thus making the spun bond polypropylene laminated onto the middle layer/wound contact layer.

Samples are cut into 10x10 cm, then packed into pouches, and sterilized by gamma irradiation. The finished dressing has a weight of 230 gsm, absorbency 30 g/100 cm2.

## Claims

1. A three-layered wound dressing wherein the dressing has a wound contact layer, a middle absorbent layer and an outer layer, the said wound contact layer comprises hydrophilic fibres and hydrophobic fibres, the middle layer is a nonwoven comprising hydrophilic fibres and hydrophobic fibres, and the outer layer is a nonwoven of hydrophobic fibres, and wherein the percentage of the said hydrophilic fibres in the wound contact layer is 75% or less by weight of the wound contact layer.

2. A three-layered wound dressing according to claim 1 wherein the said hydrophilic fibres of the wound contact layer are selected from: viscose fibres, absorbent acrylic fibres, absorbent polypropylene fibres, Lyocell fibres, alginate fibres, hydrophilic chitosan fibres, carboxymethyl cellulose (CMC) fibres, carboxy ethyl cellulose (CEC) fibres, acylated chitosan fibres, carboxymethyl chitosan fibres; the said hydrophobic fibres of the wound contact layer are selected from: polypropylene fibres, polyester fibres, nylon (Polyamide) fibres, polyethylene fibres, hydrophobic chitosan fibres, PP/PE bi-component fibres, PA/PE bi-component fibres, PET/PA bi-component fibres.

3. A three-layered wound dressing according to claim 1 wherein the middle layer comprises hydrophilic and hydrophobic fibres, and the percentage of the said hydrophobic fibres is, by the weight of the middle layer, 75% or less.

4. A three-layered wound dressing according to claim 1 wherein the said hydrophilic fibres of the middle layer are selected from viscose fibres, absorbent chitosan fibres, alginate fibres, Lyocell fibres, carboxymethyl cellulose (CMC) fibres, carboxy ethyl cellulose (CEC) fibres, acylated chitosan fibres, carboxymethyl chitosan fibres, cross-linked acrylates copolymer super absorbent fibres and wood pump fibres; the said hydrophobic fibres of the middle layer are selected from polypropylene fibres, polyester fibres, nylon (Polyamide) fibres, polyethylene fibres, hydrophobic chitosan fibres, PP/PE bi-component fibres, PA/PE bi-component fibres, PET/PA bi-component fibres.

5. A three-layered wound dressing according to claim 1 wherein the hydrophobic of the outer layer is selected from polypropylene fibres, polyester fibres, nylon (Polyamide) fibres, polyethylene fibres, hydrophobic chitosan fibres, PP/PE bi-component fibres, PA/PE bi-component fibres, PET/PA bi-component fibres.

6. A three-layered wound dressing according to claim 1 wherein the weight of the said wound contact layer is between 50-500 gram per square meter (gsm), and /or the weight of the middle layer is between 80-500 gsm and/or the weight of the outer layer is between 51-100 gsm.

7. A three-layered wound dressing according to claim 1 wherein the colour of the said wound contact layer, middle layer is undyed or dyed.

8. A three-layered wound dressing according to claim 1 wherein the hydrophilic fibres and /or hydrophobic fibres of the said wound contact layer and/or the middle layer comprises antimicrobial agents such as silver, silver compound, silver complex, polyhexamethylene biguanide (PHMB) and honey.

9. A method to manufacture the said three-layered wound dressing according to claim 1 wherein the method comprises one of the following sets of steps:
a. Manufacturing the wound contact layer, the middle absorbent layer and the outer layer separately using the heat binding method, or the needle punching method or the hydro entanglement method, then laminating the three layers together by heat bonding, or needle punching or chemical bonding, followed by cutting, packing and sterilization;
b. Manufacturing the wound contact layer and the middle absorbent layer separately using the heat binding method, or the needle punching method or the hydro entanglement method, and laminating the wound contact layer and the middle absorbent layer together by heat bonding, or needle punching or chemical bonding, then manufacture the outer layer onto the said laminated wound contact layer/middle layer directly, followed by cutting, packing and sterilization;
c. Manufacturing the middle absorbent layer and the outer layer separately using the heat binding method, or the needle punching method or the hydro entanglement method, and laminate the middle absorbent layer and the outer layer together by heat bonding, or needle punching or chemical bonding, then manufacturing the wound contact layer onto the said laminated middle layer/outer layer directly, followed by cutting, packing and sterilization.

## Patentansprüche

1. Dreischichtiger Wundverband, wobei der Verband eine Wundkontaktschicht, eine mittlere absorbierende Schicht und eine Außenschicht hat, wobei die Wundkontaktschicht hydrophile Fasern und hydrophobe Fasern umfasst, wobei die mittlere Schicht ein Vlies ist, der hydrophile Fasern und hydrophobe Fasern umfasst, und wobei die Außenschicht ein Vlies aus hydrophoben Fasern ist, und wobei der Prozentsatz der hydrophilen Fasern in der Wundkontaktschicht 75 Gewichtsprozent oder weniger der Wundkontaktschicht ist.

2. Dreischichtiger Wundverband nach Anspruch 1, wobei die hydrophilen Fasern der Wundkontaktschicht ausgewählt sind aus: Viskosefasern, absorbierenden Acrylfasern, absorbierenden Polypropylenfasern, Lyocellfasern, Alginatfasern, hydrophilen Chitosanfasern, Carboxymethyl-Zellulose- (CMC-) Fasern, Carboxyethyl-Zellulose- (CEC-) Fasern, acylierten Chitosanfasern, Carboxymethyl-Chitosanfasern; wobei die hydrophoben Fasern der Wundkontaktschicht ausgewählt sind aus: Polypropylenfasern, Polyesterfasern, Nylon- (Polyamid-) Fasern, Polyethylenfasern, hydrophobe Chitosanfasern, PP/PE-Bikomponentenfasern, PA/PE-Bikomponentenfasern, PET/PA-Bikomponentenfasern.

3. Dreischichtiger Wundverband nach Anspruch 1, wobei die mittlere Schicht hydrophile und hydrophobe Fasern umfasst, und wobei der Prozentsatz der hydrophoben Fasern 75% oder weniger des Gewichts der mittleren Schicht ist.

4. Dreischichtiger Wundverband nach Anspruch 1, wobei die hydrophilen Fasern der mittleren Schicht ausgewählt sind aus Viskosefasern, absorbierenden Chitosanfasern, Alginatfasern, Lyocellfasern, Carboxymethyl-Zellulose- (CMC-) Fasern, Carboxyethyl-Zellulose- (CEC-) Fasern, acylierten Chitosanfasern, Carboxymethyl-Chitosanfasern, quervernetzten superabsorbierenden Acrylat-Copolymerfasern und Holzzellstofffasern; wobei die hydrophoben Fasern der mittleren Schicht ausgewählt sind aus Polypropylenfasern, Polyesterfasern, Nylon- (Polyamid-) Fasern, Polyethylenfasern, hydrophoben Chitosanfasern, PP/PE-Bikomponentenfasern, PA/PE-Bikomponentenfasern, PET/PA-Bikomponentenfasern.

5. Dreischichtiger Wundverband nach Anspruch 1, wobei das Hydrophobe der äußeren Schicht ausgewählt ist aus Polypropylenfasern, Polyesterfasern, Nylon-(Polyamid-) Fasern, Polyethylenfasern, hydrophoben Chitosanfasern, PP/PE-Bikomponentenfasern, PA/PE-Bikomponentenfasern, PET/PA-Bikomponentenfasern.

6. Dreischichtiger Wundverband nach Anspruch 1, wobei das Gewicht der Wundkontaktschicht zwischen 50-500 Gramm pro Quadratmeter (gsm) ist, und/oder wobei das Gewicht der mittleren Schicht zwischen 80-500 gsm ist, und/oder wobei das Gewicht der Außenschicht zwischen 51-100 gsm ist.

7. Dreischichtiger Wundverband nach Anspruch 1, wobei die Farbe der Wundkontaktschicht, mittleren Schicht ungefärbt oder gefärbt ist.

8. Dreischichtiger Wundverband nach Anspruch 1, wobei die hydrophilen Fasern und/oder die hydrophoben Fasern der Wundkontaktschicht und/oder der mittleren Schicht antimikrobielle Mittel wie zum Beispiel Silber, eine Silberverbindung, einen Silberkomplex, Polyhexamethylen-Biguanid (PHMB) und Honig umfassen.

9. Verfahren zur Herstellung des dreischichtigen Wundverbands nach Anspruch 1, wobei das Verfahren einen von der folgenden Gruppe von Schritten umfasst:
a. Herstellen der Wundkontaktschicht, der mittleren absorbierenden Schicht und der Außenschicht separat unter Verwendung des Hitzeverbindungsverfahrens oder des Vernadelungsverfahrens oder des Wasserstrahlverfestigungsverfahrens, dann Laminieren der drei Schichten aneinander durch Hitzeverbinden oder Vernadeln oder chemisches Verbinden, gefolgt von Schneiden, Packen und Sterilisieren;
b. Herstellen der Wundkontaktschicht und der mittleren absorbierenden Schicht separat unter Verwendung des Hitzeverbindungsverfahrens oder des Vernadelungsverfahrens oder des Wasserstrahlverfestigungsverfahrens, und Laminieren der Wundkontaktschicht und der mittleren absorbierenden Schicht aneinander durch Hitzeverbinden oder Vernadeln oder chemisches Verbinden, dann Herstellen der äußeren Schicht direkt auf der laminierten Wundkontaktschicht/mittleren Schicht, gefolgt von Schneiden, Packen und Sterilisieren;
c. Herstellen der mittleren absorbierenden Schicht und der Außenschicht separat unter Verwendung des Hitzeverbindungsverfahrens oder des Vernadelungsverfahrens oder des Wasserstrahlverfestigungsverfahrens, und Laminieren der mittleren absorbierenden Schicht und der Außenschicht aneinander durch Hitzeverbinden oder Vernadeln oder chemisches Verbinden, dann Herstellen der Wundkontaktschicht direkt auf der laminierten mittleren Schicht/Außenschicht, gefolgt von Schneiden, Packen und Sterilisieren.

## Revendications

1. Pansement à trois couches dans lequel le pansement comporte une couche de contact avec la plaie, une couche absorbante centrale et une couche extérieure, ladite couche de contact avec la plaie comprend des fibres hydrophiles et des fibres hydrophobes, la couche centrale est un non-tissé comprenant des fibres hydrophiles et des fibres hydrophobes, et la couche extérieure est un non-tissé de fibres hydrophobes, et dans lequel le pourcentage desdites fibres hydrophiles dans la couche de contact avec la plaie est de 75 % ou moins en poids de la couche de contact avec la plaie.

2. Pansement à trois couches selon la revendication 1 dans lequel lesdites fibres hydrophiles de la couche de contact avec la plaie sont sélectionnées parmi : des fibres de viscose, des fibres d'acrylique absorbantes, des fibres de polypropylène absorbantes, des fibres de lyocell, des fibres d'alginate, des fibres de chitosane hydrophiles, des fibres de carboxyméthylcellulose (CMC), des fibres de carboxyéthylcellulose (CEC), des fibres de chitosane acylé, des fibres de chitosane de carboxyméthyle ; lesdites fibres hydrophobes de la couche de contact avec la plaie sont sélectionnées parmi : des fibres de polypropylène, des fibres de polyester, des fibres de nylon (polyamide), des fibres de polyéthylène, des fibres de chitosane hydrophobes, des fibres à deux composants PP/PE, des fibres à deux composants PA/PE, des fibres à deux composants PET/PA.

3. Pansement à trois couches selon la revendication 1 dans lequel la couche centrale comprend des fibres hydrophiles et hydrophobes, et le pourcentage desdites fibres hydrophobes est, en poids de la couche centrale, de 75 % ou moins.

4. Pansement à trois couches selon la revendication 1 dans lequel lesdites fibres hydrophiles de la couche centrale sont sélectionnées parmi des fibres de viscose, des fibres de chitosane absorbantes, des fibres d'alginate, des fibres de lyocell, des fibres de carboxyméthylcellulose (CMC), des fibres de carboxyéthylcellulose (CEC), des fibres de chitosane acylé, des fibres de chitosane de carboxyméthyle, des fibres super-absorbantes de copolymère d'acrylates réticulés et des fibres de pâte de bois ; lesdites fibres hydrophobes de la couche centrale sont sélectionnées parmi des fibres de polypropylène, des fibres de polyester, des fibres de nylon (polyamide), des fibres de polyéthylène, des fibres de chitosane hydrophobes, des fibres à deux composants PP/PE, des fibres à deux composants PA/PE, des fibres à deux composants PET/PA.

5. Pansement à trois couches selon la revendication 1 dans lequel la fibre hydrophobe de la couche extérieure est sélectionnée parmi des fibres polypropylène, des fibres de polyester, des fibres de nylon (polyamide), des fibres de polyéthylène, des fibres de chitosane hydrophobes, des fibres à deux composants PP/PE, des fibres à deux composants PA/PE, des fibres à deux composants PET/PA.

6. Pansement à trois couches selon la revendication 1 dans lequel le poids de ladite couche de contact avec la plaie est compris entre 50 et 500 grammes par mètre carré (gsm), et/ou le poids de la couche centrale est compris entre 80 et 500 gsm et/ou le poids de la couche extérieure est compris entre 51 et 100 gsm.

7. Pansement à trois couches selon la revendication 1 dans lequel la couleur de ladite couche de contact avec la plaie, de la couche centrale est non teintée ou teintée.

8. Pansement à trois couches selon la revendication 1 dans lequel les fibres hydrophiles et/ou fibres hydrophobes de ladite couche de contact avec la plaie et/ou de la couche centrale comprennent des agents antimicrobiens tels que de l'argent, un composé d'argent, un complexe d'argent, un biguanide de polyhexaméthylène (PHMB) et du miel.

9. Procédé de fabrication dudit pansement à trois couches selon la revendication 1 dans lequel le procédé comprend l'un des ensembles d'étapes suivants :
a. la fabrication de la couche de contact avec la plaie, de la couche absorbante centrale et de la couche extérieure séparément en utilisant le procédé de liaison thermique, ou le procédé de perforation à l'aiguille ou le procédé d'hydro-enchevêtrement, puis la stratification des trois couches ensemble par liaison thermique, ou perforation à l'aiguille ou liaison chimique, suivie par la découpe, le conditionnement et la stérilisation ;
b. la fabrication de la couche de contact avec la plaie et de la couche absorbante centrale séparément en utilisant le procédé de liaison thermique, ou le procédé de perforation à l'aiguille ou le procédé d'hydro-enchevêtrement, et la stratification de la couche de contact avec la plaie et de la couche absorbante centrale ensemble par liaison thermique, ou perforation à l'aiguille ou liaison chimique, puis la fabrication de la couche extérieure sur lesdites couche de contact avec la plaie/couche centrale stratifiées directement, suivie par la découpe, le conditionnement et la stérilisation ;
c. la fabrication de la couche absorbante centrale et de la couche extérieure séparément en utilisant le procédé de liaison thermique, ou le procédé de perforation à l'aiguille ou le procédé d'hydro-enchevêtrement, et la stratification de la couche absorbante centrale et de la couche extérieure ensemble par liaison thermique, ou perforation à l'aiguille ou liaison chimique, puis la fabrication de la couche de contact avec la plaie sur lesdites couche centrale/couche extérieure stratifiées directement, suivie par la découpe, le conditionnement et la stérilisation.
